# EUROPEAN PATENT APPLICATION

(11) **EP 0 769 297 A1**
(43) Date of publication of application: **23.04.1997**
(21) Application number: 96307521.3
(22) Date of filing: 16.10.1996
(51) Int. Cl.: A61K 31/495

(54) **Use of nefazodone for the manufacture of a medicament for the treatment of panic attack**

(30) Priority: 17.10.1995 US 5688
(71) Applicant: BRISTOL-MYERS SQUIBB COMPANY, New York, N.Y. 10154-0037 (US)
(72) Inventor: Marcus, Ronald, Hamden, CT 06518 (US); Kensler, Terry T., Madison, CT 06443 (US)
(74) Representative: Jones, Alan John

(57) **Abstract**

Nefazodone and its pharmaceutically acceptable salts are useful in alleviation of panic disorders which can take the form of clinical syndromes comprising, for example, panic attacks, agoraphobia and phobic anxiety.

## Description

This invention is concerned with a drug bio-affecting body-treating process which employs the compound 2-[3-[4-(3-chlorophenyl)-1-piperazinyl]propyl]-5-ethyl-2,4-dihydro-4-(2-phenoxyethyl)-3H-,2,4-triazol-3-one or a pharmaceutically acceptable acid addition salt thereof.

This compound has the following structural formula (I) 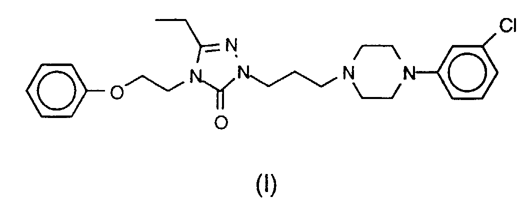 and is known as nefazodone. The hydrochloride salt has been referred to in the literature as MJ 13754-1 and as BMY 13754, as well as nefazodone hydrochloride, which is the United States Adopted Name (USAN); refer to "USP Dictionary of USAN and International Drug Names," 1995, p. 459.

Synthesis of nefazodone and close analogs and disclosure of its pharmacology are described in the following patents and publications.
1. Temple, et al, U.S. 4,338,317 issued July 6, 1982.
2. Gammans, U.S. 5,116,852, issued May 26, 1992.
3. D.P. Taylor, et al, "Nefazodone Hydrochloride," Drugs of the Future, 12(8) pp. 758-759 (1987).
4. A. Eison, et al, "Nefazodone: Preclinical Pharmacology of a New Antidepressant," Psychopharmacology Bulletin, 26(3) pp. 311,315 (1990).

Clinical studies of nefazodone have indicated its usefulness as an antidepressant agent and nefazodone hydrochloride has been approved by the U.S. Food and Drug Administration for use in treating depressed patients. Nefazodone also appears to have sleep normalizing properties in a human population. This contrasts with effects on sleep seen for other antidepressant drugs.

The method of the present invention can be distinguished from the above prior art in that it is directed to a distinct patient population characterized by a disease state different from that related to depression disclosed in this prior art. Support for this distinction is found in "The Diagnostic Validity of Anxiety Disorders and Their Relationship to Depressive Illness," by A.B. Boyer, et al, in Am. J. Psychiatry, 142:7, pp. 787-796 (1985).

Although compared to depression panic disorder is a relatively new diagnosis, the basic diagnostic concepts are well known to those skilled in the art and are clearly differentiated from generalized, persistent anxiety states. The following references are examples of literature reviewing the diagnosis and treatment of panic disorders.
- American Psychiatric Association, Diagnostic and Statistical Manual of Mental Disorders, Fourth Edition, pp. 394-403 (1994).
- M. Balestrieri, et al, "Drug Treatment of Panic Disorder - A Critical Review of Controlled Trials," Psychiatric Developments, 4, pp. 337-350 (1989).
- J. Coplan, et al, "Serotonin Related Functions in Panic-Anxiety: A Critical Overview," Neuropsychopharmacology, 6(3) pp. 189-200 (1992).
- H. Westenberg, et al, "Serotonin-lnfluencing Drugs in the Treatment of Panic Disorder," Psychopathology, 22 (suppl. 1) pp. 68-77, (1989).
- D. Sheehan, et al, "Panic Disorder: The Potential Role of Serotonin Reuptake Inhibitors," Journal of Clinical Psychiatry, 49 (8 suppl.) pp. 30-36 (1988).

While the use of nefazodone for treating panic disorder is novel, other antidepressant agents have been disclosed to be effective in the treatment of panic disorder. There is, however, no direct connection between antipanic activity and antidepressant activity, as some potent antidepressants, such as Buproprion, have not been shown to have antipanic activity. The differences in the efficacy of agents acting at different points in the serotonin system may be attributed to the pathophysiology of panic disorder. It is possible that nefazodone possesses important clinical distinctions from other available treatments, due to its dual effects on serotonergic transmission (i.e., 5-HT₂ antagonism and 5-HT uptake inhibition) and norepinephrine uptake.

The most relevant antidepressant drug for comparison to nefazodone would seem to be trazodone, with its structure (II) containing a meta-chlorophenylpiperazine moiety 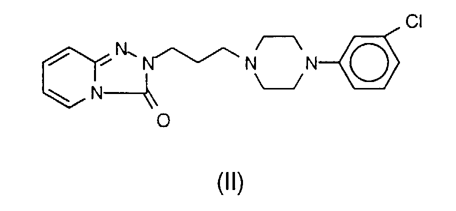 similar to nefazodone. While an early clinical report by Marissakalian, et al, (Am. J. Psychiatry, 1987, 144:785-787) of an open study concluded that nefazodone may have specific antipanic action, a clinical report (Chamey, et al, J. Clin. Psychiatry, 1986, 47:580-6) of a double blinded, controlled, comparative drug trial concluded that trazodone, in contrast to the imipramine and alprazolam, was not an effective treatment for panic disorder. Other reports have cast doubt on trazodone being effective in panic disorder and a clinical review (Marek, et al, Psychopharmacology, 1992, 109:2-11) concludes that trazodone does not appear to be effective in treating obsessive compulsive disorder (OCD) and panic disorder.

We believe that upon consideration of all applicable prior art there is no teaching or suggestion that nefazodone would be useful in alleviation of panic disorder.

The process of the present invention is intended for the alleviation of panic disorders of which panic attacks, agoraphobia, and phobic anxiety are specific symptoms. The process essentially involves administration of nefazodone, or a pharmaceutically acceptable acid addition salt thereof, to one in need of such treatment. For use in the instant process, oral administration of nefazodone hydrochloride from about 300 to 500 mg per day in divided doses is anticipated as being the preferred dosage regimen. While various treatments have been employed to treat panic disorder, it is possible that nefazodone possesses important clinical distinctions from other available treatments due to its dual effects on serotonergic transmission and norepinephrine uptake. A data analysis of 55 depressed patients with co-morbid panic disorder revealed that 100 to 500 mg doses of nefazodone produced significant improvement in panic symptoms as compared to imipramine and placebo.

Figure 1 graphically demonstrates the time course relationship over a six-week treatment period of mean change in patient panic factor scores for placebo, imipramine, and nefazodone hydrochloride treated patient groups.

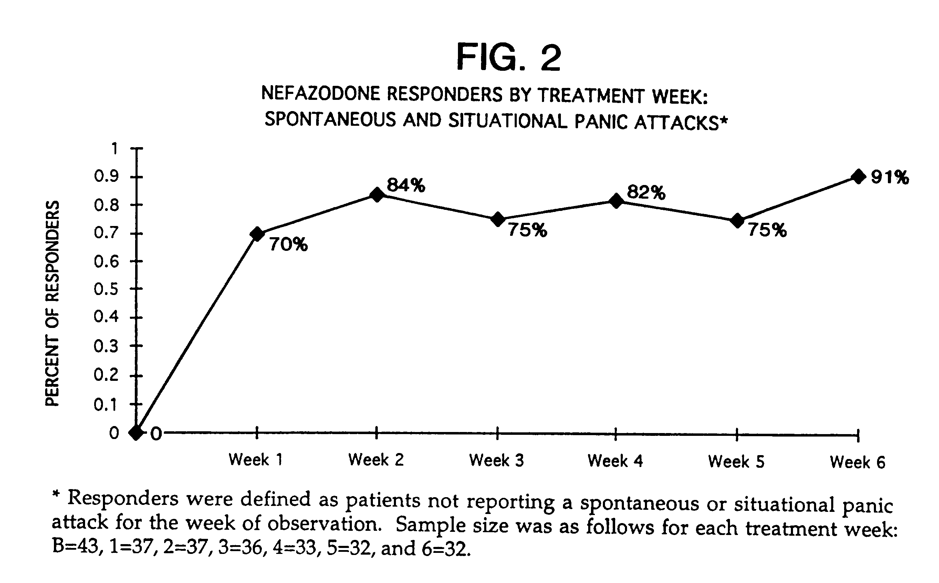

Figure 2 shows the time course relationship for 43 nefazodone-treated patients experiencing panic attacks (prior to study randomization) over a six-week treatment period.

Panic disorders are best defined clinically by the frequent occurrence of panic attacks in patients. A panic attack is described as a sudden surge of intense discomfort and/or fear which can occur either spontaneously, seemingly without cause, or can occur as situational episodes. Within ten minutes of the onset of the panic attack, a variety of characteristic symptoms may develop. These symptoms can include shortness of breath, choking or smothering sensations, palpitations or accelerated heart rate, chest pain, sweating, faintness, dizziness, light-headedness, nausea or abdominal distress, depersonalization or derealization, numbness or tingling sensations, hot flashes or chills, trembling or shaking, a fear of dying, or a fear of becoming insane or losing mental control. The frequency and severity of these attacks can result in phobic anxiety and behavior which, in certain instances, can cause the patient to be housebound, or severely restricted in social behavior.

To date, various treatments have been employed for treatment of patients suffering from panic disorders. These treatments include hypnosis and behavior therapies as well as pharmacotherapy. Imipramine hydrochloride and phenelzine sulfate have been widely prescribed drugs for this indication and, although effective for relief or panic attacks, have undesirable side effects which limit their usefulness. While Alprazolam is approved in the United States for use in treating panic disorder, in general clinical results with benzodiazepines appear to be variable.

It has now been found that nefazodone alleviates some of the symptoms associated with panic disorders. This finding was made by analysis of changes in symptoms characteristic of panic disorder, contained as items in standard psychometric instruments. To illustrate, a subanalysis of data for 55 depressed patients with comorbid panic disorder was evaluated over a six-week treatment period using panic and phobic anxiety factors which were extracted from the Symptom Check List (SCL-90). The SCL-90 is a self-report symptom inventory, consisting of 90 items, which can be factored into clinical clusters with diagnostic utility; cf: Wilson, et al, British Joumal of Psychiatry, 147, pp. 400-403 (1985), and references therein. The high dose nefazodone group (100 to 500 mg daily) produced significant improvement in panic-related symptoms as compared to imipramine and placebo. Along with marked improvement in panic symptoms, there was also a trend toward statistical significance for phobic anxiety symptoms to improve after six weeks of nefazodone at higher doses; see Table 1.

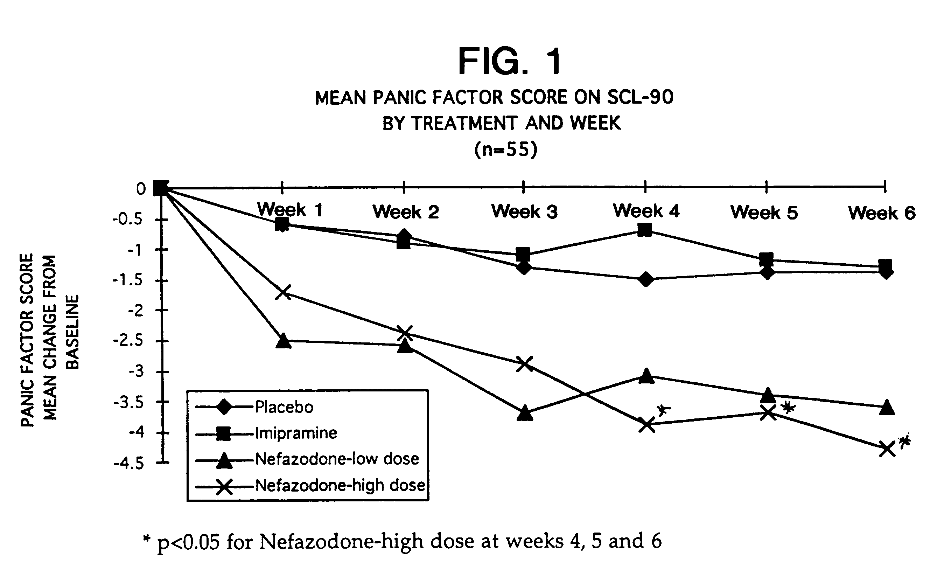

Figure 1 shows the time course relationship of the mean change from baseline of the patient panic factor score by drug treatment group. The panic factor score is obtained for each patient at baseline and weeks 1 through 6 by summing the numerical values assigned to each panic factor symptom item according to severity and/or frequency being experienced. The higher the patient score, the greater the degree of illness. As can be seen, there is greater improvement in mean change from baseline for the nefazodone group compared with the imipramine and placebo groups. The comparative improvement for the nefazodone group becomes much more evident after week 2 of the study. There was no significant difference in baseline scores for the treatment groups.

A similar pattern of therapeutic oucome was seen in a meta-analysis of data from three two-arm clinical trials comparing nefazodone with other antidepressants. For the nefazodone treated patients, 81% of those diagnosed with panic attacks were symptom free (no panic attacks) at the study endpoint.

Currently, studies are being planned to continue the evaluation of nefazodone's utility in the treatment of panic disorder.

The method of the present invention essentially involves administration of nefazodone, or a pharmaceutically acceptable acid addition salt thereof, to a patient in need of such treatment. Pharmaceutically acceptable acid addition salts of nefazodone and methods of pharmaceutical formulation are described in the patent of Temple, et al, U.S. 4,338,317, which is incorporated herein in its entirety by reference.

Administration of nefazodone hydrochloride according to the present invention may be by the parenteral, oral, or rectal routes. The oral route is preferred, however. The clinical dosage for alleviation of panic disorders is expected to be around 400 mg per day, generally in the 200 to 600 mg range and preferable in the range of 300 to 500 mg per day. Since the dosage should be tailored to the individual patient, the usual practice is to commence with a dose of about 50 mg administered two times per day and then to increase the dose every week by 50 to 100 mg at each dosage time until the desired response is observed or until the patient exhibits side effects. Single daily dosage may be applicable in some instances.

**Table 1**

| Six-Week Change from Baseline Results on SCL-90 Panic¹ and Phobic Anxiety Scores | | | | |
|---|---|---|---|---|
| | | | Nefazodone | |
| Variable | Placebo (n=13) | Imipramine² (n=12) | Low Dose³ (n=15) | High Dose⁴ (n=15) |
| SCL-90 | | | | |
| Panic Score | -1.4 | -1.3 | -3.6* | -4.3* |
| Phobic Anxiety | -0.8 | -2.6 | -3.7 | -5.3* |
| Mean Modal Dose | 10 Caps | 202 mg | 243 mg | 460 mg |

| | | | | |
|---|---|---|---|---|
| * (p<0.10) ** (p<0.01) | | | | |
| ¹Panic items are "suddenly scared for no reason," "feeling fearful," and "spells of terror and panic." | | | | |
| ²Imipramine dose range was between 50-250 mg daily. | | | | |
| ³Low dose nefazodone ranged between 50-250 mg daily. | | | | |
| ⁴High dose nefazodone ranged between 100-500 mg daily. | | | | |

## Claims

1. A method for alleviation of panic disorders which comprises administering a non-toxic therapeutically effective dose of nefazodone or a pharmaceutically acceptable acid addition salt thereof to a patient in need of such treatment.

2. The method of claim 1 wherein nefazodone hydrochloride is employed and dosage is by the oral route.

3. The method of claim 1 wherein panic attacks is the specific panic disorder afflicting said patient.

4. The method of claim 1 wherein agoraphobia is the specific panic disorder afflicting said patient.

5. The method of claim 1 wherein phobic anxiety is the specific panic disorder afflicting said patient.

6. The method of claim 2, 3, 4, or 5 wherein said patient is an adult and a daily dose of from about 200 mg to 600 mg is employed.

7. The method of claim 6 wherein said daily dose is divided and administered b.i.d.

8. Use of nefazodone or a pharmaceutically acceptable acid addition salt thereof for the manufacture of a medicament for alleviation of panic disorders.

9. Use according to claim 8 of nefazodone hydrochloride.

10. Use according to claim 8 or claim 9 employing the recited feature(s) of any one or more of claims 2 to 7.
